# EUROPEAN PATENT APPLICATION

(11) **EP 0 847 991 A1**
(43) Date of publication of application: **17.06.1998**
(21) Application number: 96120182.9
(22) Date of filing: 16.12.1996
(51) Int. Cl.: C07D 207/44, C07D 209/48, C07D 209/76

(54) **Method for the preparation of N-(carboxyalkyl) imides**

(71) Applicant: DAINIPPON INK AND CHEMICALS, INC., Itabashi-ku Tokyo (JP)
(72) Inventor: Frings, Rainer B., Dr., 12307 Berlin (DE); Grahe, Gerwald F., Dr., 14195 Berlin (DE)
(74) Representative: Kraus, Walter, Dr.

(57) **Abstract**

A method is described for the preparation of N-(carboxyalkyl)imides corresponding to the general formula (I) 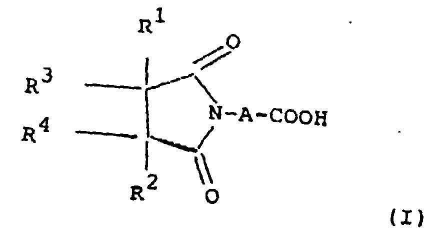 by the reaction of cyclic anhydrides corresponding to the general formula (II) 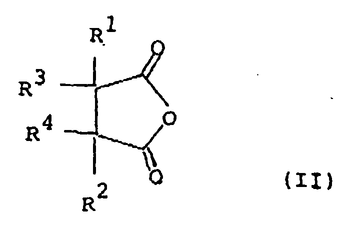 with an α,ω-aminoalkylcarboxylic acid corresponding to the general formula (III)

H₂N-A-COOH (III).

The method provides an industrially easily practicable synthesis, from inexpensive and easily available starting compounds, of N-(carboxyalkyl)imides possessing a terminal acid function and having alkyl chains containing 1 to 18 carbon atoms.

## Description

The invention relates to a method for the preparation of N-(carboxyalkyl)imides. These compounds are monomers for acid-functional polymers and are starting products, on the bioconjugate principle, for pharmaceutical active ingredients.

N-(carboxyalkyl)maleimides in particular are interesting compounds. They are used in experimental pharmacology and in molecular biology in the form of their esters with biologically active alcohols or with fluorescing compounds, the high reactivity of the maleimide double bond being utilised here primarily with thiol groups, in order to produce bioconjugates or to label specific active sites in tissues. They also render possible the preparation of acid-functional copolymers as epoxy cross-linking agents and as water-soluble and water-dispersible resins. The free choice of the alkyl chain length and hence of the distance of the acid function from the polymer chain and the high resistance to hydrolysis of the imide groups in the polymers are advantageous. N-(carboxyalkyl)maleimides of this kind having alkyl chain lengths of longer than 5 C atoms can also be used as polymerisable emulsifiers in emulsion polymerisations.

Although, as expressed above, N-(carboxyalkyl)imides are interesting preliminary products for the preparation of polymers and pharmaceuticals, their industrial use has hitherto been frustrated by their poor availability.

The hitherto known syntheses of N-(carboxyalkyl)imides, for example, of N-(carboxyalkyl)maleimides, have been described by O. Keller and J. Rudinger in Helvetica Chimica Acta, 56, No. 63, page 531 ff. (1975), by J. Rich et al. in J. of Medicinal Chemistry 18 (10), page 1004 ff. (1975) and by T. Saegusa et al. in Macromolecules 23, page 2636 ff. (1990). In the first method, maleimide is converted into the desired N-(carboxyalkyl)imides in a three-stage reaction via N-alkoxycarbonylmaleimides, subsequent reaction with aminocarboxylic acids and basic cyclisation. Here the reaction is commenced using the costly starting product maleimide and the further reaction steps require the precise maintenance of specific pH values and reaction times in order to achieve optimal yields. This method cannot be carried out for the industrial production of polymerisable imide monomers.

The second method starts from maleic anhydride and amino acids, which in an initial step are reacted in acetic acid to form the corresponding maleimido acids which, after purification, are cyclised under reflux with triethylamine in toluene to form maleimides. The N-(carboxyalkyl)maleimides are released from the triethylammonium salts isolated by acidification with HCl.

In the third method, which describes the preparation of 3-maleimidopropionic acid, the maleamidopropionic acid initially formed in THF at room temperature is isolated and then cyclised at 70^{o}C in an acetic anhydride/sodium acetate mixture. The authors give the yield as 13% of theoretical yield, after costly purification. This method follows the synthesis of N-arylmaleimides, which results here however in considerably higher yields.

Although the second method is a simplification compared with the synthetic route according to Keller and Rudinger, it is unsatisfactory as an industrial solution because of the two reaction steps, the required purification of the intermediate product and the use of different solvents. Moreover a considerable quantity of not easily separable diethylamides of the corresponding N-(carboxyalkyl)maleimides is formed when this synthetic route is adopted.

The third method is also unsuitable for industrial syntheses, primarily on account of the low yield and the costly purification.

The object forming the basis of the present invention is accordingly to overcome the disadvantages of the above-mentioned known methods for the preparation of N-(carboxyalkyl)imides and to provide a synthesis which can be easily carried out industrially, whereby N-(carboxyalkyl)imides possessing a terminal acid function and having alkyl chains containing 1 to 18 carbon atoms can be prepared from inexpensive and easily available starting compounds.

This object is fulfilled by the present invention.

The invention is based on the surprising finding that N-(carboxyalkyl)imides, contrary to previous information contained in the literature, can be prepared in a one-step synthesis directly from the corresponding cyclic anhydride and a linear or branched α,ω-aminoalkylcarboxylic acid, when the said reactants are reacted with one another, with dehydration, in an aliphatic carboxylic acid having a boiling point between 100 and 200°C, at the reflux temperature of the carboxylic acid.

The invention therefore provides a method for the preparation of N-(carboxyalkyl)imides corresponding to the general formula (I) 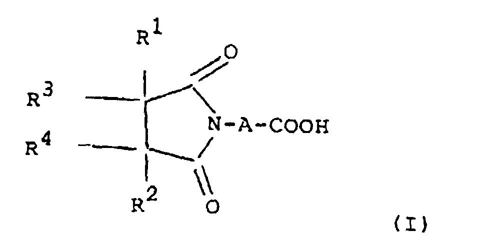 wherein R¹ and R² independently of one another represent hydrogen, halogen, C₁-C₄ alkyl or phenyl, R³ and R⁴ either together form a double bond or, together with the carbon atoms to which they are bonded, form a fused norbornene, cyclohex-4-ene or benzene ring, which may carry one or more additional substituents, and in the case that R³ and R⁴ together with the carbon atoms to which they are bonded form a fused benzene ring, the groups R¹ and R² are not present, and A signifies the group

-CH₂-[CR⁵R⁶]ₙ₋,

wherein R⁵ and R⁶ independently of one another represent hydrogen, alkyl or phenyl, with the total number of carbon atoms being 18 at most, and n having the value 0 to 16,
which is characterised in that a cyclic anhydride corresponding to the general formula (II) 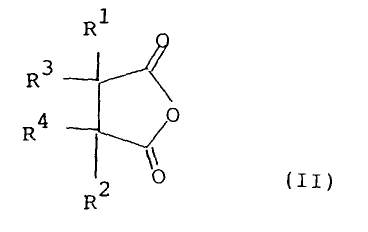 wherein R¹, R², R³ and R⁴ are defined as above, together with from 0.8 to 1.2 mol of a linear or branched α,ω-aminoalkylcarboxylic acid corresponding to the general formula (III)

H₂N-A-COOH (III),

wherein A has the meaning given above, are dehydrated in an aliphatic carboxylic acid having a boiling point between 100 and 200°C, at the reflux temperature of the carboxylic acid.

In the general formula (I), R¹ and R² independently of one another represent a hydrogen atom or halogen atom, for example, a fluorine, chlorine or bromine atom, preferably a chlorine atom, a C₁-C₄ alkyl group, preferably a methyl or ethyl group, or a phenyl group, R³ and R⁴ either together form a double bond or, together with the carbon atoms to which they are bonded, form a fused norbornene, cyclohex-4-ene or benzene ring, which may carry one or more substituents. Where R³ and R⁴ together with the carbon atoms to which they are bonded form a fused benzene ring, the groups R¹ and R² are absent. The N-(carboxyalkyl)imides may therefore also be represented by the general formula (Ia) 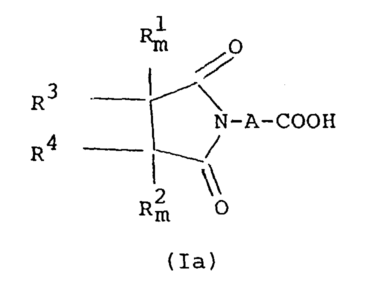 wherein R¹, R², R³, R⁴ and A are defined as above, and in the case that R³ and R⁴ together with the carbon atoms to which they are bonded form a benzene ring, m has the value 0 and in all other cases has the value 1. When unsubstituted or substituted maleic anhydride is used as cyclic anhydride, the N-(carboxyalkyl)maleimides prepared according to the invention can be represented by the following formula (Ib) 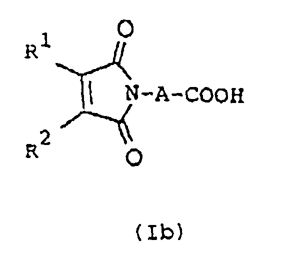 wherein R¹ and R² are defined as above.

In the case of a fused norbornene ring, the N-(carboxyalkyl)imides prepared according to the invention may be represented by the following formula (Ic) 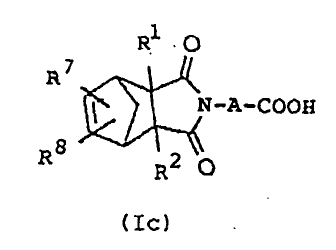 wherein R¹ and R² are defined as above, and R⁷ and R⁸ independently of one another represent hydrogen, halogen, C₁-C₄ alkyl or phenyl.

In the case of a cyclohex-4-ene ring, the N-(carboxyalkyl)imides prepared according to the invention may be represented by the following formula (Id) 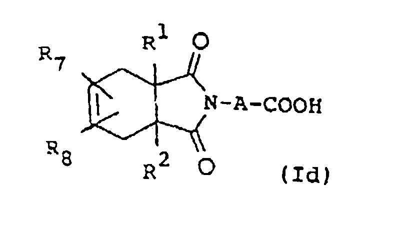 wherein R¹ and R² are defined as above, and R⁷ and R⁸ independently of one another represent hydrogen, halogen, C₁-C₄ alkyl or phenyl.

In the case of a benzene ring, the N-(carboxyalkyl)imides prepared according to the invention may be represented by the following formula (Ie) 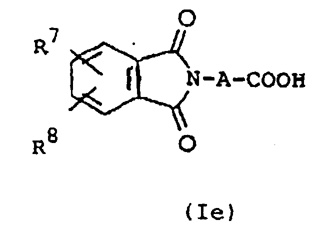 wherein R⁷ and R⁸ independently of one another represent hydrogen, halogen, C₁-C₄ alkyl or phenyl and the benzene ring may additionally contain one or two further substituents.

The above N-(carboxyalkyl)imides may carry one or more, for example, two or three, substituents, preferably one or two substituents. Possible substituents are in particular halogen atoms, for example, fluorine, chlorine or bromine atoms, preferably chlorine or bromine atoms, and also C₁-C₄ alkyl groups, preferably methyl or ethyl groups, as well as the phenyl group.

A forms the group

-CH₂-[CR⁵R⁶]ₙ₋,

wherein R⁵ and R⁶ independently of one another represent a hydrogen atom, an alkyl group, preferably a C₁-C₄ alkyl group, in particular a methyl or ethyl group, or a phenyl group. The total number of carbon atoms in the group A is 18 at most, preferably 1 to 11; n is an integer within the range 0 to 16, preferably within the range 0 to 10.

In the method according to the invention, a cyclic anhydride corresponding to the general formula (II) 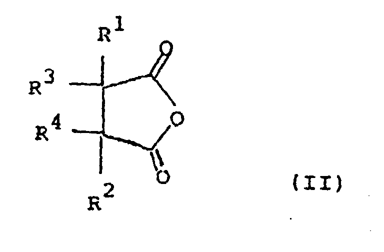 wherein R¹, R², R³ and R⁴ are defined as above, is reacted with a linear or branched α,ω-aminoalkylcarboxylic acid corresponding to the general formula (III)

H₂N-A-COOH (III),

wherein A has the meaning given above. The α,ω-aminoalkylcarboxylic acid has 2 to 18 carbon atoms, preferably 2 to 12 carbon atoms. Suitable α,ω-aminocarboxylic acids are, for example, 2-aminoacetic acid, 3-aminopropionic acid, 4-aminobutyric acid, 3-aminoisobutyric acid, 6-aminohexanoic acid (aminocaproic acid), 8-aminooctanoic acid, 10-aminodecanoic acid, 11-aminoundecanoic acid, 12-aminododecanoic acid and 18-aminostearic acid, as well as the monoalkylated and polyalkylated derivatives thereof such as, for example, aminopivalic acid. By means of the method according to the invention α,ω-aminoalkylcarboxylic acids having chirality centres in the methylene chain can also be converted, without racemisation, into optically active imidocarboxylic acids. Examples of this are D- and L-3-aminoisobutyric acid. Aminoalkylcarboxylic acids wherein the primary amino group is a substituent of a tertiary carbon atom are less preferred.

The reaction takes place in an aliphatic carboxylic acid having a boiling point between 100 and 200°C, at the reflux temperature of the carboxylic acid. Examples of suitable carboxylic acids are formic acid, acetic acid, propionic acid, n-butyric and isobutyric acid. Acetic acid is preferred. The reaction takes place preferably in a fivefold to tenfold molar excess of the concentrated liquid carboxylic acid. The starting materials, that is, the cyclic anhydride corresponding to the general formula (II) and the α,ω-aminoalkylcarboxylic acid corresponding to the general formula (III), are usefully dissolved or suspended, with stirring, in the concentrated liquid carboxylic acid. The reaction takes place at the reflux temperature of the particular carboxylic acid. The duration of the reaction time is, for example, 3 to 18 hours, preferably 5 to 12 hours. Optionally 0.05 wt.% of an acid catalyst, preferably sulphuric acid, concentrated phosphoric acid, p-toluenesulphonic acid, or a strongly acidic ion-exchange resin such as Amberlyst A15®, may be added to the reaction mixture. The coreactants are used in quantities such that 1 mol of cyclic anhydride is reacted with 0.8 to 1.2 mol of the linear or branched α,ω-aminoalkylcarboxylic acid For example, the reaction may be carried out using equimolar proportions, but the use of a 10 to 20% molar excess of either component is also possible. In one preferred embodiment of the method according to the invention, from 5 to 50 wt.%, preferably from 10 to 25 wt.%, referred to the carboxylic acid, of a solvent which forms an azeotrope with water is also added to the reaction mixture in order to remove from the mixture the water of reaction formed, Toluene and mixtures of isomeric xylenes are preferred azeotrope-forming solvents. This procedure permits the determination of the end point of the reaction from the quantity of water formed, lessens the formation of secondary products, and shortens the reaction times through the continuous removal of the water from the equilibrium. After the reaction has been carried out, the reaction mixture is worked up in the conventional manner, for example, by filtering off insoluble precipitates, distilling off the solvent mixture in a vacuum and recrystallising the residue.

A preferred embodiment of the method according to the invention is characterised in that the cyclic anhydride used is a maleic anhydride corresponding to the general formula (IV) 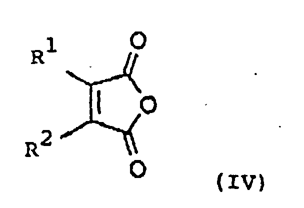 wherein R¹ and R² are defined as above.

Examples of suitable maleic anhydrides are maleic anhydride, 2-chloro-, 2,3-dichloro-, 2-methyl- and 2,3-dimethylmaleic anhydrides.

Another preferred embodiment of the method according to the invention is characterised in that the cyclic anhydride used is a 5-norbornene-2,3-dicarboxylic anhydride corresponding to the general formula (V) 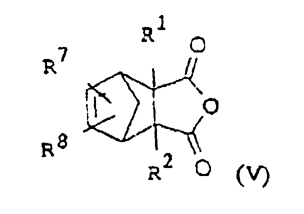 wherein R¹ and R² are defined as above, and R⁷ and R⁸ independently of one another represent hydrogen, halogen, C₁-C₄ alkyl or phenyl.

Examples of suitable norbornene dicarboxylic anhydrides are 5-norbornene-2,3-dicarboxylic anhydride, methyl-5-norbornene-2,3-dicarboxylic anhydride isomeric mixtures, 2-methyl-5-norbornene-2,3-dicarboxylic anhydride and 2,3-dimethyl-5-norbornene-2,3-dicarboxylic anhydride.

Another preferred embodiment of the method according to the invention is characterised in that the cyclic anhydride used is a 1,2,3,6-tetrahydrophthalic anhydride corresponding to the general formula (VI) 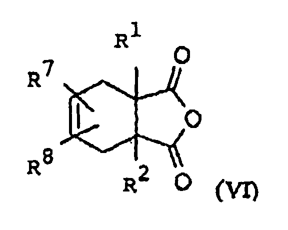 wherein R¹ and R² are defined as above, and R⁷ and R⁸ independently of one another represent hydrogen, halogen, C₁-C₄ alkyl or phenyl.

Examples of suitable tetrahydrophthalic anhydrides are 1,2,3,6-tetrahydrophthalic anhydride, 3-methyl-1,2,3,6-tetrahydrophthalic anhydride, 4-methyl-1,2,3,6-tetrahydrophthalic anhydride, 4-phenyl-1,2,3,6-tetrahydrophthalic anhydride and 4-chloro-1,2,3,6-tetrahydrophthalic anhydride.

Finally, a further preferred embodiment of the method according to the invention is characterised in that the cyclic anhydride used is a phthalic anhydride corresponding to the general formula (VII) 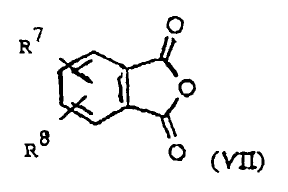 wherein R⁷ and R⁸ independently of one another represent hydrogen, halogen, C₁-C₄ alkyl or phenyl, and which may additionally contain one or two further substituents.

Examples of suitable phthalic anhydrides are phthalic anhydride, 3-nitrophthalic anhydride, 4-nitrophthalic anhydride, 3-phenylphthalic anhydride, 4-phenylphthalic anhydride and benzene-1,2,4-tricarboxylic anhydride (trimellitic anhydride).

When phthalic, norbornene and tetrahydrophthalic anhydrides are used, the yields are higher than those obtained when unsubstituted and substituted maleic anhydrides are used, as no secondary reactions such as, for example, Michael additions, can take place with the inactivated double bonds of these anhydrides. In most cases the use of an acid catalyst can also be dispensed with here.

The N-(carboxyalkyl)imides obtained by the method according to the invention can be converted by known methods into further derivatives, such as acid chlorides and esters. They are therefore valuable and easily available starting products and intermediate products for the preparation of pharmaceutical active ingredients, diagnostic reagents, monomers and natural substances. They can moreover be converted by homopolymerisation and copolymerisation into polymers having high temperature resistance, high mechanical strength and very good resistance to chemicals, the latter attributable to the high stability to acids and bases of the polymerised imide groups. The N-(carboxyalkyl)imides are polymerised by means of conventional polymerisation methods.

The invention is explained in the Examples.

### Examples

### Example 1

150.0 g (2 mol) of aminoacetic acid (glycine) and 196.0 g (2 mol) of maleic anhydride are suspended in a mixture of 2.5 l of anhydrous acetic acid and 300 ml of toluene and heated to reflux temperature in a reaction apparatus equipped with a Dean-Stark water trap. After the addition of 25 ml of concentrated sulphuric acid, 43 ml of a water/acetic acid mixture is removed within 16 hours. After the mixture has been cooled, undissolved precipitate is removed by filtration and the solvent mixture is distilled off in a vacuum. The residue is recrystallised from water and yields 218 g of maleimidoacetic acid (glycine maleimide), which corresponds to 70.3% of the theoretical yield. The melting point of 113 to 114^{o}C and the IR, NMR and mass spectra correspond to the data in the technical literature.

### Example 2

178.0 g (2 mol) of 3-aminopropionic acid (β-alanine) and 196.0 g (2 mol) of maleic anhydride are suspended in a mixture of 2.5 l of anhydrous acetic acid and 300 ml of toluene and heated to reflux temperature in a reaction apparatus equipped with a Dean-Stark water trap. In the course of this a clear solution is formed. After the addition of 0.5 ml of concentrated sulphuric acid, 40 ml of a water/acetic acid mixture is removed within 5 hours. The solvent mixture is then distilled off in a vacuum and the residue is recrystallised from diethyl ether and dried. 281 g of 3-maleimidopropionic acid is formed, which corresponds to 75% of the theoretical yield. The melting point of 95 to 98^{o}C and the IR, NMR and mass spectra correspond to the data in the technical literature.

### Example 3

206.0 g (2 mol) of 4-aminobutyric acid and 196.0 g (2 mol) of maleic anhydride are suspended in a mixture of 2.5 l of anhydrous acetic acid and 300 ml of toluene and heated to reflux temperature in a reaction apparatus equipped with a Dean-Stark water trap. In the course of this a clear solution is formed. After the addition of 0.5 ml of concentrated sulphuric acid, 180 ml of a water/acetic acid mixture is removed within 6 hours. After the mixture has been cooled, the solvent mixture is distilled off in a vacuum. The highly viscous residue is recrystallised from chloroform/petroleum ether (5:1 v/v) and yields 117 g of 4-maleimidobutyric acid, which corresponds to 29% of the theoretical yield. The melting point of 68 to 73^{o}C and the IR, NMR and mass spectra correspond to the data in the technical literature.

### Example 4

60.0 g (0.61 mol) of 6-aminohexanoic acid and 80.4 g (0.61 mol) of maleic anhydride are dissolved in 400 ml of anhydrous acetic acid and boiled under reflux for 5 hours, with the formation of a clear solution. After the mixture has been cooled, the solvent mixture is distilled off in a vacuum and the solid residue is recrystallised from 600 ml of water. After drying, 50.1 g of 6-maleimidohexanoic acid, corresponding to 41% of the theoretical yield, is obtained. The melting point of 83 to 85^{o}C and the IR, NMR and mass spectra correspond to the data in the technical literature.

### Example 5

302.0 g (1.5 mol) of 11-aminoundecanoic acid and 147.0 g (1.5 mol) of maleic anhydride are suspended in a mixture of 2.5 l of anhydrous acetic acid and 300 ml of toluene and heated to reflux temperature in a reaction apparatus equipped with a Dean-Stark water trap. In the course of this a clear solution is formed. After the addition of 5 ml of concentrated sulphuric acid, 103 ml of a water/acetic acid mixture is removed within 12 hours. After the mixture has been cooled, the solvent mixture is distilled off in a vacuum. The wax-like residue is recrystallised from 1.8 l of ethyl acetate and yields 212 g of 11-maleimidoundecanoic acid, which corresponds to 50% of the theoretical yield. The melting point of 77 to 82^{o}C and the IR, NMR and mass spectra correspond to the data in the technical literature.

### Example 6

32.8 g (0.2 mol) of 5-norbornene-2,3-dicarboxylic anhydride and 26.2 g (0.2 mol) of 6-aminohexanoic acid are dissolved in a mixture of 300 ml of anhydrous acetic acid and 50 ml of toluene and heated at reflux temperature for 5.5 hours in a reaction apparatus equipped with a Dean-Stark water trap. In the course of this 11 ml of a water/acetic acid mixture is removed. After the mixture has been cooled, the solvent mixture is distilled off in a vacuum. There remains 52.6 g, equal to 95% of the theoretical yield, of a colourless viscous oil, whereof the NMR, IR and mass spectra correspond to those of pure 6-nadimidohexanoic acid.

### Example 7

32.8 g (0.20 mol) of 5-norbornene-2,3-dicarboxylic anhydride and 40.3 g (0.20 mol) of 11-aminoundecanoic acid are dissolved in a mixture of 300 ml of acetic acid and 50 ml of toluene and heated to reflux temperature in a reaction apparatus equipped with a Dean-Stark water trap. In the course of this a clear solution is rapidly formed. Within 5 hours, 15 ml of a water/acetic acid mixture is removed. After the mixture has been cooled, the solvent mixture is distilled off in a vacuum, leaving behind a highly viscous oil, which slowly crystallises out. The crystallisate is isolated by vacuum filtration, washed with 400 ml of petroleum ether and dried at 50^{o}C in a vacuum. 60.2 g, corresponding to 90% of the theoretical yield, of a product is formed, which is shown by its NMR spectra to be pure 11-nadimidoundecanoic acid and has a melting point of 67 to 71^{o}C.

### Example 8

33.3 g (0.2 mol) of 4-methyl-1,2,3,6-tetrahydrophthalic anhydride and 26.2 g (0.2 mol) of 6-aminohexanoic acid are dissolved in a mixture of 300 ml of anhydrous acetic acid and 50 ml of toluene and heated at reflux temperature for 5.5 hours in a reaction apparatus equipped with a Dean-Stark water trap. In the course of this 11 ml of a water/acetic acid mixture is removed. After the mixture has been cooled, the solvent mixture is distilled off in a vacuum There remains 49.8 g, equal to 89% of the theoretical yield, of a colourless viscous oil, whereof the NMR, IR and mass spectra correspond to those of pure 4-methyltetrahydrophthalimido-6-hexanoic acid.

### Example 9

13.1 g (0.1 mol) of 6-aminohexanoic acid and 19.2 g (0.1 mol) of trimellitic anhydride are dissolved in a mixture of 150 ml of anhydrous acetic acid and 20 ml of toluene and heated under reflux for 2.5 hours. In the course of this a clear solution is rapidly formed and 5.3 ml of a water/acetic acid mixture is removed. After the mixture has been cooled, the crystallisate is isolated by vacuum filtration, washed with about 1 l of petroleum ether and dried at 50^{o}C in a vacuum. 25.8 g, corresponding to 84% of the theoretical yield, of product is formed, which is shown by its NMR, IR and mass spectra to be pure trimellitimido-6-hexanoic acid and has a melting point of 205 to 209^{o}C.

### Example 10

13.1 g (0.1 mol) of 6-aminohexanoic acid and 19.3 g (0.1 mol) of 3-nitrophthalic anhydride are dissolved in a mixture of 150 ml of anhydrous acetic acid and 20 ml of toluene and heated under reflux for 4.5 hours. In the course of this a clear solution is rapidly formed and 6 ml of a water/acetic acid mixture is removed. After the reaction mixture has been cooled on an ice/common salt mixture, the product slowly crystallises out, is isolated by vacuum filtration, washed with about 300 ml of petroleum ether and dried at 50^{o}C in a vacuum. 22.9 g, corresponding to 75% of the theoretical yield, of a product is formed, which is shown by its NMR, IR and mass spectra to be pure 3-nitrophthalimido-6-hexanoic acid and has a melting point of 151 to 153^{o}C.

## Claims

1. A method for the preparation of N-(carboxyalkyl)imides corresponding to the general formula (I) 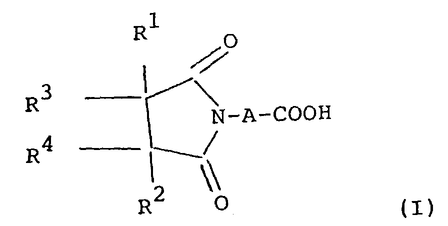 wherein R¹ and R² independently of one another represent hydrogen, halogen, C₁-C₄ alkyl or phenyl, R³ and R⁴ either together form a double bond or, together with the carbon atoms to which they are bonded, form a fused norbornene, cyclohex-4-ene or benzene ring, which may carry one or more additional substituents, and in the case that R³ and R⁴ together with the carbon atoms to which they are bonded form a fused benzene ring, the groups R¹ and R² are not present, and A signifies the group
-CH₂-[CR⁵R⁶]ₙ₋,
wherein R⁵ and R⁶ independently of one another represent hydrogen, alkyl or phenyl, with the total number of carbon atoms being 18 at most, and n having the value 0 to 16,
which is characterised in that a cyclic anhydride corresponding to the general formula (II) 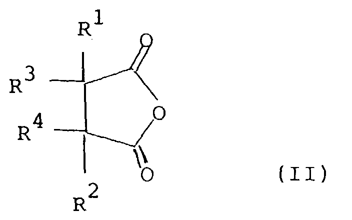 wherein R¹, R², R³ and R⁴ are defined as above, together with from 0.8 to 1.2 mol of a linear or branched α,ω-aminoalkylcarboxylic acid corresponding to the general formula (III)
H₂N-A-COOH (III),
wherein A has the meaning given above, are dehydrated in an aliphatic carboxylic acid having a boiling point between 100 and 200°C, at the reflux temperature of the carboxylic acid.

2. The method according to claim 1, characterised in that the cyclic anhydride used is a maleic anhydride corresponding to the general formula (IV) 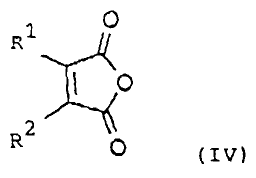 wherein R¹ and R² are defined as in claim 1.

3. The method according to claim 1, characterised in that the cyclic anhydride used is a 5-norbornene-2,3-dicarboxylic anhydride corresponding to the general formula (V) 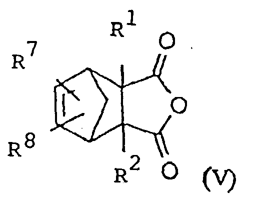 wherein R¹ and R² are defined as in claim 1, and R⁷ and R⁸ independently of one another represent hydrogen, halogen, C₁-C₄ alkyl or phenyl.

4. The method according to claim 1, characterised in that the cyclic anhydride used is a 1,2,3,6-tetrahydrophthalic anhydride corresponding to the general formula (VI) 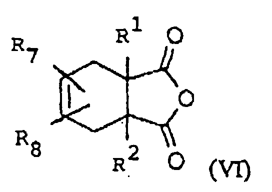 wherein R¹ and R² are defined as in claim 1, and R⁷ and R⁸ independently of one another represent hydrogen, halogen, C₁-C₄ alkyl or phenyl.

5. The method according to claim 1, characterized in that the cyclic anhydride used is a phthalic anhydride corresponding to the general formula (VII) 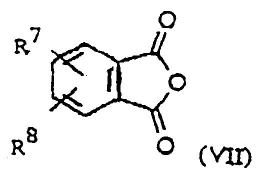 wherein R⁷ and R⁸ independently of one another represent hydrogen, halogen, C₁-C₄ alkyl or phenyl, and which may additionally contain one or two further substituents.

6. The method according to one of claims 1 to 5, characterised in that the aliphatic carboxylic acid is used in a fivefold to tenfold molar excess with respect to the total quantity of the cyclic anhydride and of the α,ω-aminoalkylcarboxylic acid.

7. The method according to one of claims 1 to 6, characterised in that the imidisation reaction is carried out in the presence of from 0.1 to 5 wt.% of a strongly acid catalyst, referred to the total quantity of the cyclic anhydride and of the α,ω-aminoalkylcarboxylic acid.

8. The method according to one of claims 1 to 7, characterised in that the imidisation reaction is carried out in the presence, referred to the aminoalkylcarboxylic acid component, of from 5 to 50 wt.% of an azeotrope-forming solvent, with continuous removal of the water of reaction.

9. The use of an N-(carboxyalkyl)imide prepared by the method according to claims 1 to 8 as a starting product for polymers.
